# EUROPEAN PATENT APPLICATION

(11) **EP 2 138 149 A1**
(43) Date of publication of application: **30.12.2009**
(21) Application number: 07827985.8
(22) Date of filing: 05.11.2007
(51) Int. Cl.: A61J 1/05, A61M 5/30

(54) **AMPULE GUIDE AND MEDICAL SOLUTION DOSING UNIT**

(30) Priority: 07.11.2006 JP 2006302171
(71) Applicant: Oyama, Yoshio, Kanagawa 216-0035 (JP)
(72) Inventor: Oyama, Yoshio, Kanagawa 216-0035 (JP)
(74) Representative: Schweiger, Martin
(86) International application number: PCT/JP2007/001207
(87) International publication number: WO 2008/056443

(57) **Abstract**

An object of the present invention is to provide an ampoule guide and a drug solution administering unit having excellent stability. The above object is solved by an ampoule guide (30) which is provided with a hollow cylindrical guard (31) at a tipping portion and which accommodates therein an ampoule (20); a drug solution administering unit provided with an ampoule guide (30) which is provided with a hollow cylindrical guard (31) at a tipping portion and which accommodates therein an ampoule (20), a gasket (40) which is set within the ampoule (20) for accommodating and pushing out a drug solution, and a plunger activating portion (50) provided with a push-out mechanism for pushing out the gasket (40); and the like.

## Description

### [Technical Field]

The present invention relates to an ampoule guide and a drug solution administering unit. More specifically, the present invention relates particularly to an ampoule guide, a drug solution administering unit and the like enabling a cartridge type ampoule usable as a needleless syringe to be utilized effectively.

### [Background Art]

Generally, many of drugs are orally administered. However, as for insulin for treating diabetes, interferon for treating hepatitis C or cancer, and the like, a patient himself or herself administers the drug using an injection syringe. Treating methods using such a form of administration require continuous administration of injectable solution. Therefore, the patient has daily suffered from fear of the syringe needle, ache upon administration of the injectable solution, or the like, resulting in great mental burden.

Therefore, needleless syringes with no needles have been developed and used in recent years as substitutes for syringes with needles. A needleless syringe shoots the drug solution at an extremely high speed towards the skin. As a result, a small hole is made at the skin, so that the drug solution penetrates through the skin. Accordingly, the drug solution can be administered (see for example, Japanese Patent Application Laid-Open Publication No. 2003-093508 (Patent Citation 1)).

However, every time the above-mentioned needleless syringe is used, it is required first to attach a special adapter to a vial. After a specified quantity of the drug solution is suctioned from the vial into the syringe barrel, this syringe barrel having suctioned the drug solution is reattached to an injector. Thereafter, the syringe is applied to a specified site for a shot, where the drug solution is pushed out at a high speed to be administered. Accordingly, the handling is complicated and it is not always easy to suction and administer an adequate amount. Moreover, since the same needleless syringe is used multiple times for suctioning the drug solution for administration, problems exist from a hygienic perspective such that viruses and the like may attach to the aperture plane and replicate. Therefore, preparation of hermetically sealed drug solution in an amount per single administration to be opened and administered per administration can be conceived.

[Patent Citation 1] Japanese Patent Application Laid-Open Publication No. 2003-093508

### [Disclosure of Invention]

### [Problems to be solved by the Invention]

An object of the present invention is to provide an ampoule guide and a drug solution administering unit that are excellent in stability when administering a drug with an ampoule.

### [Means for solving the Problems]

The present invention is based on a knowledge that basically by providing a hollow cylindrical guard (31) at a tipping portion of an ampoule guide (30) which accommodates an ampoule (20), the guard (31) acts as a leg, so that an orientation of the ampoule is secured, thereby providing a drug solution administering unit which is excellent in stability when administering a drug with an ampoule.

The first aspect of the present invention relates to an ampoule guide (30) comprising: a hollow cylindrical guard (31) which is provided at a tipping portion, the ampoule guide being capable of accommodating therein an ampoule (20).

A preferred embodiment of the first aspect of the present invention relates to an ampoule guide, wherein when the ampoule (20) is inserted into the ampoule guide (30), a tip of the hollow cylindrical guard (31) is located on an inner side with respect to an open end of the ampoule (20).

The second aspect of the present invention relates to a drug solution administering unit comprising: an ampoule (20); an ampoule guide (30) which is provided with a hollow cylindrical guard (31) at a tipping portion and which accommodates therein the ampoule (20) ; and a gasket (40) which is set within the ampoule (20) for accommodating and pushing out a drug solution. It is to be noted that when the present invention is used as a needleless syringe unit, a plunger activating portion (50) equipped with a push-out mechanism for pushing out the gasket (40) may be provided as appropriate.

A preferred embodiment of the second aspect of the present invention relates to the drug solution administering unit as described above, wherein the ampoule (20) comprises: a front edge portion (1) which is removed when the ampoule (20) is used; a main body (2) which can accommodate a drug solution; a connection (3) which connects the front edge portion (1) and the main body (2); and a joint (4) which joins the front edge portion (1) and the connection (3), and which is narrower than the front edge portion (1) and the connection (3); the front edge portion(1), the joint (4), the connection (3) and the main body (2) being coaxially aligned to have a central axis (5), having sequential space portions (11, 14, 13, 12) extending from the main body (2) to a midway of the front edge portion (1), the joint (4) breaking when a force is applied from a lateral direction with respect to the central axis (5), so that a head portion of the connection (3) which is circular truncated cone shaped is exposed and the space portion (13) of the connection (3) becomes an open end. In the expression "a front edge portion which is removed when the ampoule is used", "when the ampoule is used" means when drug solution or the like is administered using the ampoule.

A preferred embodiment of the second aspect of the present invention relates to the above-mentioned drug solution administering unit, wherein when the ampoule (20) is inserted into the ampoule guide (30), a tip of the hollow cylindrical guard (31) is located on an inner side with respect to an open end of the ampoule (20).

A preferred embodiment of the second aspect of the present invention relates to any one of the above-mentioned drug solution administering units, wherein the ampoule guide (30) is provided with one or more notched parts (32) in a direction parallel to the central axis (5).

A preferred embodiment of the second aspect of the present invention relates to any one of the above-mentioned drug solution administering units, wherein a plurality of projections (33) is provided in a direction parallel to the central axis (5), with projections that are taller than their surrounding projections among the plurality of projections being provided at angular intervals of any one of 60 degrees, 90 degrees, 120 degrees and 180 degrees.

### [Effects of the Invention]

According to the present invention, a hollow cylindrical guard (31) acts as a leg at a tipping portion of an ampoule guide (30) which accommodates an ampoule (20). Therefore, an orientation of the ampoule is secured, so that it is made possible to provide a drug solution administering unit which is excellent in stability when administering a drug with an ampoule.

### [Brief Description of the Drawings]

[Fig. 1] Fig. 1 is a configuration diagram of a drug solution administering unit of the present invention.
[Fig. 2] Fig. 2 is a diagram showing an ampoule guide of the present invention.
[Fig. 3] Fig. 3 is a schematic diagram of a drug solution administering unit of the present invention.
[Fig. 4] Fig. 4 is a diagram showing a basic structure of an ampoule of the present invention.
[Fig. 5] Fig. 5 is a diagram showing an ampoule after use.
[Fig. 6] Fig. 6 is a schematic diagram showing a drug solution administering unit of the present invention as pressed against a skin.
[Fig. 7] Fig.7 is a schematic diagram showing a drug solution administering unit of the present invention as pressed against a skin in a horizontal direction.

### [Explanation of Reference]

- 1: front edge portion
- 2: main body
- 3: connection
- 4: joint
- 5: central axis
- 11-14: space portion
- 20: ampoule
- 30: ampoule guide
- 31: hollow cylindrical guard
- 40: gasket
- 50: plunger activating portion

### [Best Mode for Carrying Out the Invention]

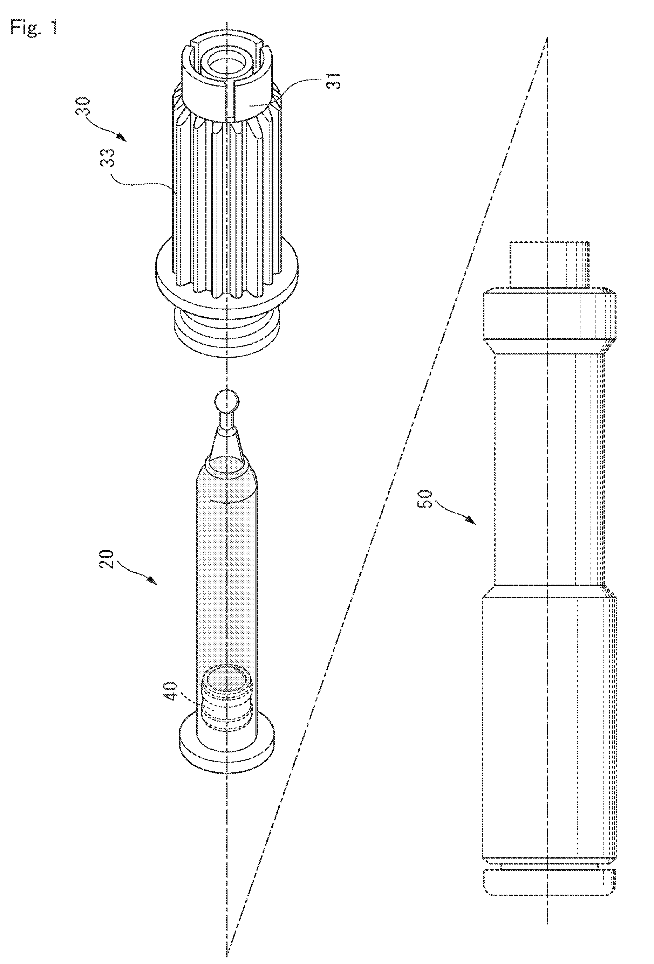

Hereinafter, the present invention will be described according to the drawings. Fig. 1 is a configuration diagram of a drug solution administering unit of the present invention. As shown in Fig. 1, the drug solution administering unit is provided with an ampoule (20); an ampoule guide (30) which is provided with a hollow cylindrical guard (31) at a tipping portion and which accommodates therein the ampoule (20), and a gasket (40) which is set within the ampoule (20) for accommodating and pushing out a drug solution. Since the hollow cylindrical guard (31) acts as a leg at the tipping portion of the ampoule guide (30) which accommodates the ampoule (20), an orientation of the ampoule is secured, so that it is made possible to provide a drug solution administering unit which is excellent in stability when administering a drug with an ampoule. It is to be noted that a cartridge type ampoule having accommodated a drug solution within the ampoule (20) in advance and stopped with the gasket (40) in that state is preferable.

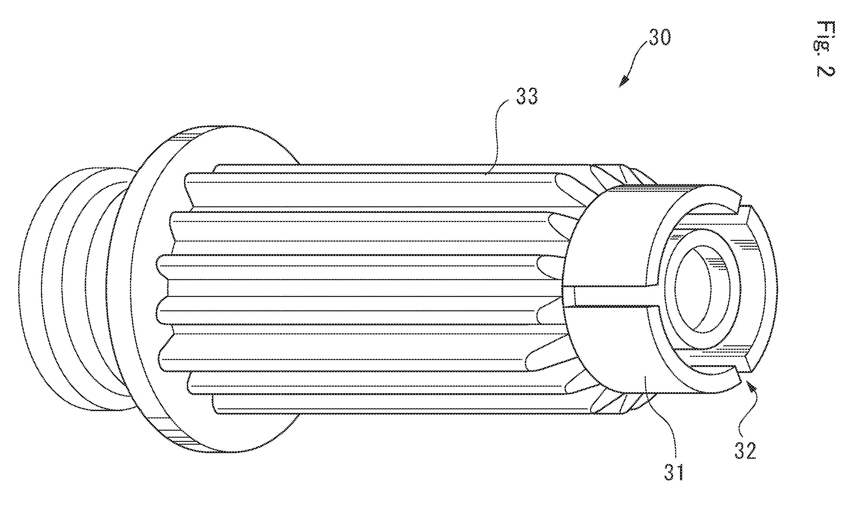

Fig. 2 is a diagram showing an ampoule guide of the present invention. As shown in Fig. 2, the present invention relates to an ampoule guide (30) provided with a hollow cylindrical guard (31) at a tipping portion and which accommodates therein the ampoule (20). With a preferred embodiment of this ampoule guide, when the ampoule (20) is inserted into the ampoule guide (30), a tip of the hollow cylindrical guard (31) is located on an inner side with respect to an open end (a front end portion of a connection (3)) of the ampoule (20). The ampoule guide of the present invention is preferably provided with one or more notched parts (32) in a direction parallel to the central axis (5). Such notched parts (32) provide ways of escape for the flesh when the drug solution administering unit is pressed against the skin, so that the stability of the drug solution administering unit is increased.

From the view point of effectively letting the flesh under the skin escape, between 0.5 mm and 5 mm, both inclusive, can be mentioned as widths of the notched parts (32), while between 1 mm and 3 mm, both inclusive is acceptable. In case of providing a plurality of notched parts (32), between 2 and 12, both inclusive, can be mentioned as the number of the notched parts (32), while between 3 and 6, both inclusive, is acceptable. Also, in case of providing a plurality of notched parts (32), from the view point of stability, it is preferable that the notched parts (32) are equally spaced.

Also, when the ampoule guide (30) is attached to the drug solution administering unit, the ampoule guide is rotated in a state where the ampoule is included therein. However, the ampoule guide (30) is slippery since it is formed of metal such as aluminum, plastic or the like. Accordingly, attaching an ampoule guide is not always easy for a child or a woman who is not so strong. Therefore, it is preferable that a plurality of projections (33) is provided in a direction parallel to the central axis (5), with projections that are taller than their surrounding projections among the plurality of projections being provided at angular intervals of any one of 60 degrees, 90 degrees, 120 degrees and 180 degrees. Such an ampoule guide (30) can be rotated easily with a small power to be attached to the drug solution administering unit since the projections and the tall projections act as a grip.

A preferred embodiment of the first aspect of the present invention relates to the above-mentioned drug solution administering unit, wherein the ampoule (20) is provided with: a front edge portion (1) which is removed when the ampoule (20) is used; a main body (2) which can accommodate a drug solution; a connection (3) which connects the front edge portion (1) and the main body (2); and a joint (4) which joins the front edge portion (1) and the connection (3), and which is narrower than the front edge portion (1) and the connection (3); the front edge portion (1), the joint (4), the connection (3) and the main body (2) are coaxially aligned to have a central axis (5), having sequential space portions (11, 14, 13, 12) extending from the main body (2) to a midway of the front edge portion (1), and the joint (4) breaks when a force is applied from a lateral direction with respect to the central axis (5), so that a head portion of the connection (3) which is circular truncated cone shaped is exposed and the space portion (13) of the connection (3) becomes an open end.

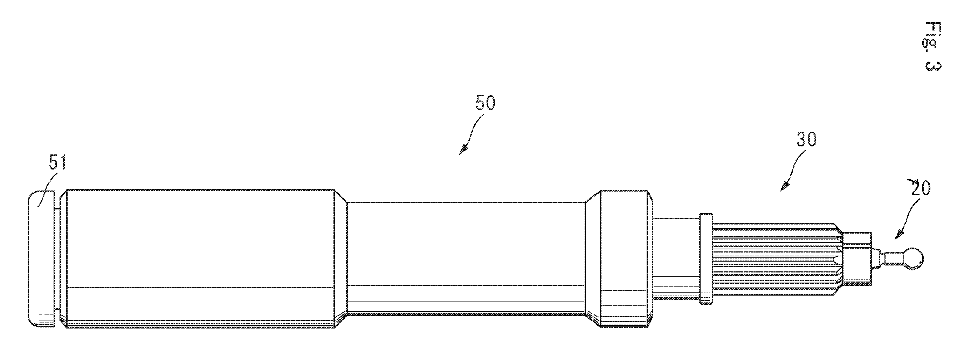

Fig. 3 is a schematic diagram of a drug solution administeringunit of the present invention. Namely, the ampoule guide (30) is rotated in a state where the ampoule (20) is included therein, so that a screw provided at the bottom of the ampoule guide is fitted in a groove provided on an inner surface of a front edge portion of a plunger activating portion.

As shown in Fig. 3, the preferred embodiment of the first aspect of the present invention relates to the above-mentioned drug solution administering unit, wherein when the ampoule (20) is inserted into the ampoule guide (30), a tip of the hollow cylindrical guard (31) is located on an inner side with respect to the front end portion of the connection (3). Generally, when the drug solution administering unit is thus arranged, the opening of the ampoule will project forward from the front end portion of the hollow cylindrical guard (31) to touch the skin, so that it is made possible to precisely control the releasing direction of the drug solution within the ampoule.

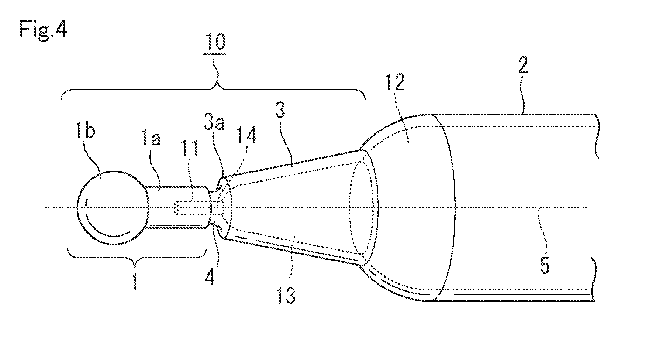

Fig. 4 is a diagram showing a basic structure of the ampoule of the present invention. As shown in Fig. 4, the ampoule (20) used for the present invention is provided with: a front edge portion (1) which is removed when the ampoule (20) is used; a main body (2) which can accommodate a drug solution; a connection (3) which connects the front edge portion (1) and the main body (2); and a joint (4) which joins the front edge portion (1) and the connection (3) and which is narrower than the front edge portion (1) and the connection (3). The front edge portion (1), the joint (4), the connection (3) and the main body (2) are coaxially aligned to have a central axis (5), having sequential space portions (11, 14, 13, 12) extending from the main body (2) to a midway of the front edge portion (1). The joint (4) breaks when a force is applied from a lateral direction with respect to the central axis (5), so that a head portion of the connection (3) which is circular truncated cone shaped is exposed and the space portion (13) of the connection (3) becomes an open end.

More specifically, as an ampoule used for the present invention, an ampoule provided with a front edge portion (1); a circular truncated cone shaped connection (3) which connects the front edge portion (1) and the main body (2) ; and a joint (4) which joins the front edge portion (1) and the connection (3) and which is narrower than the front edge portion (1) and the connection (3) can be mentioned. The front edge portion(1), the joint (4), the connection (3) and the main body (2) are coaxially aligned to have a central axis (5), having sequential space portions (11, 14, 13, 12) extending from the main body (2) to a midway of the front edge portion (1), or sequential space portions (14, 13, 12) extending from the main body (2) to a midway of the joint (4). The joint (4) breaks when a force is applied from a lateral direction with respect to the central axis (5), so that a head portion of the circular truncated cone shaped connection (3) is exposed and the space portion (13) of the connection (3) becomes an open end.

Since the connection (3) is circular truncated cone shaped and the width of the joint (4) is narrowed, the joint can easily break by simply applying a lateral force to the front edge portion (1), and the broken surface becomes nearly planar. Thus, a smooth and flat open end can be quickly and easily obtained. The space portion (14) of the joint (4) and the space portion (11) of the front edge portion (1) are preferably cylindrical, circular truncated cone shaped, or inverse circular truncated cone shaped which is coaxial to the central axis (5), and 1×10⁻¹ mm - 1 mm can be mentioned as its average diameter, while 1×10⁻¹ mm - 5×10⁻¹ is more preferable. Depth of entry or range of diffusion of the drug solution can be controlled by changing the shape or the average diameter.

The front end portion is used by breaking the joint so as to obtain the open end. Therefore, sequential space portions (14, 13, 12) extending from the main body (2) to the midway of the joint (4) may be provided. However, considering the ease of manufacturing the hermetical container end, it is preferable to provide sequential space portions (11, 14, 13, 12) extending from the main body (2) to a midway of the front edge portion (1). In Fig. 4, the space portion (11) of the front edge portion and the space portion (14) of the joint are made into a sequential cylindrical portion, which may be shaped narrower towards the tip. As an angle of inclination for this narrowing (i.e. apex angle of the cone made by extending the portion which becomes narrower towards the tip), 1 degree - 45 degrees can be mentioned, while 5 degrees - 30 degrees is preferable, and 10 degrees - 20 degrees is more preferable, and 5 degrees - 10 degrees is also applicable. Such an angle enables adjustment of liquid current so as to increase the entry speed of the drug solution and deliver the drug to a deeper portion under the skin. On the other hand, the width may be made wider towards the tip, in which case, the angle may be reversed from the above-mentioned angle. In such a case, the entry speed of the liquid is slowed down as a result, but the liquid can be diffused to be administered to a relatively wide range. It is to be noted that the space portion (13) of the connection (3) becomes narrower towards the tip, and the angle (apex angle) may be made along the outer shape of the connection (3). This will enable the strength to be uniform. Also, the space portion of the connection may be intentionally designed to have a different shape from the outer shape of the connection, for example, a portion shaped narrower towards the tip. As an angle of inclination for a portion thus shaped, 1 degree - 45 degrees can be mentioned, while 5 degrees - 30 degrees is preferable, 10 degrees - 20 degrees is more preferable, and 5 degrees - 10 degrees is also applicable.

A preferred embodiment of the hermetical container end according to the first aspect of the present invention is the above-mentioned hermetical container, wherein the front edge portion (1) is provided with a barrel portion (1a) having a cylindrical contour which is connected to the joint (4) and a head portion (1b), which is provided at a tip of the barrel portion, having a spherical contour or a cylindrical contour with a larger diameter than that of the barrel portion (1a).

Since a large head portion (1b) is thus provided at the front edge portion (1), by pressing the head portion (1b) and by applying the "principle of leverage", a great power can be applied to the joint (4). Therefore, the bonding face can break easily, and a smooth and flat open end can be quickly and easily obtained by applying a lateral torque. It is to be noted that if the length of the front edge portion along the axis is too short, it becomes difficult to apply a lateral force, while if the length is too long, it becomes less portable. Accordingly, for the length of the front edge portion 3 mm - 5×10 mm can be mentioned, while 5 mm - 1×10 mm is more preferable. Also, it is preferable that the space portion (11) connected to the space portion (14) of the joint is provided to a midway of the barrel portion (1a) within the front edge portion (1). Due to such a space portion (11), an opening can be obtained when the joint breaks.

Another preferred embodiment of hermetical container end according to the first aspect of the present invention is any one of the above-mentioned hermetical container ends, wherein an average outer diameter of the joint (4) is 1/4 - 1/2, preferably 1/3 - 1/2, of a head portion (3a) of the connection (3).

Since the average outer diameter of the joint (4) is within the above-mentioned range, the joint can easily break when a force is applied to the front edge portion.

Another preferred embodiment of hermetical container end according to the first aspect of the present invention is any one of the above-mentioned hermetical container ends, wherein the head portion (3a) of the connection has a planar portion which vertically intersects with the central axis (5). It is to be noted that the head portion (3a) may be tapered. For such a tapered portion, a height of 1×10⁻¹ mm - 5×10⁻¹ mm, preferably 2×10⁻¹ mm - 4×10⁻¹ mm, can be mentioned, a base diameter of 1 mm - 5x10 mm, preferably 2 mm - 2×10 mm and more preferably 3 mm - 1×10 mm, can be mentioned. Thus, the adhesiveness between the needleless injector and the skin can be increased. It is to be noted that the tapered portion may be provided at a top face of the connection or may be a portion of the connection which is projecting from the ampoule guide (30) when the ampoule (20) is attached to the ampoule guide as shown in Fig. 3. The tapered portion means a portion such as a circular truncated cone shaped portion which is tapered towards the end.

Since the head portion (3a) of the connection has the planar portion which vertically intersects with the central axis (5), when the joint (4) breaks, it breaks along this planar portion, resulting in a flatter broken surface while preventing a situation where broken pieces are generated. It is to be noted that this planar portion is preferably made coaxial with the central axis (5).

Another preferred embodiment of hermetical container end according to the first aspect of the present invention is any one of the above-mentioned hermetical container ends, wherein the length of the joint (4) in the direction of the central axis (5) is 1×10⁻³ mm - 1 mm.

When the length of the joint is too long, the broken surface will not be flat, and a possibility of generating broken pieces becomes high. On the other hand, when the length of the joint is too short, it becomes hard to provide the joint. Therefore, for the length of the connection, 1×10⁻³ mm - 1 mm can be mentioned, while 1×10 ³ mm - 1×10⁻² mm is preferable, and 5×10⁻³ mm - 1×10⁻² mm is more preferable.

While not specifically shown, the first aspect of the present invention may be an ampoule (20) provided with: a front edge portion (1) which is removed when the ampoule (20) is used; a main body (2) which can accommodate a drug solution; a connection (3) which connects the front edge portion (1) and the main body (2); and a joint (4) which joins the front edge portion (1) and the connection (3) and which is narrower than the front edge portion (1) and the connection (3); the front edge portion(1), the joint (4), the connection (3) and the main body (2) being coaxially aligned to have a central axis (5), having sequential space portions (11, 14, 13, 12) extending from the main body (2) to a midway of the front edge portion (1); and the joint (4) breaking when a force is applied from a lateral direction with respect to the central axis (5), so that a head portion of the connection (3) which is circular truncated cone shaped is exposed and the space portion (13) of the connection (3) becomes an open end.

An ampoule is expected to have drug solution contained in its main body (2) . Therefore, it has been a common practice to integrally form an ampoule. The present invention defies such a common practice by separately forming the hermetical container end and the main body to be assembled to form a single ampoule. This enables the hermetical container end to be given a property of fragility by using, for example, a highly crystalline resin or glass. On the other hand, the main body can be effectively prevented from cracking or breaking by using relatively elastic resin.

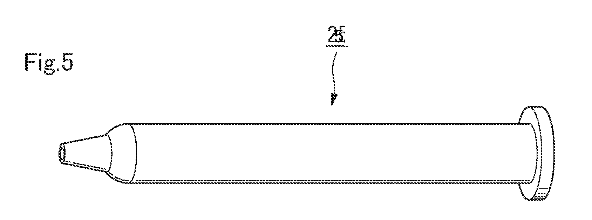

Fig. 5 is a diagram showing an ampoule after use. As shown in Fig. 5, the joint (4) breaks, so that the head portion of the connection (3) which is circular truncated cone shaped is exposed and the space portion (13) of the connection (3) becomes an open end.

As shown in Fig. 3, the plunger activating portion (50) is provided with a button (51), which is pressed to release a spring within the syringe main body, so that a movable stopper moves to release the drug solution.

Another preferred embodiment of a hermetical container end of the present invention is any one of the above-mentioned hermetical container ends, wherein the front edge portion (1), the joint (4) and the connection (3) are integrally formed, and comprised by a resin composition represented by the following general formula (I) wherein the number average molecular weight is 1×10³ - 1×10⁶ (5×10³ - 5×10⁵ is preferable, 1×10⁴ - 1×10⁵ is more preferable).

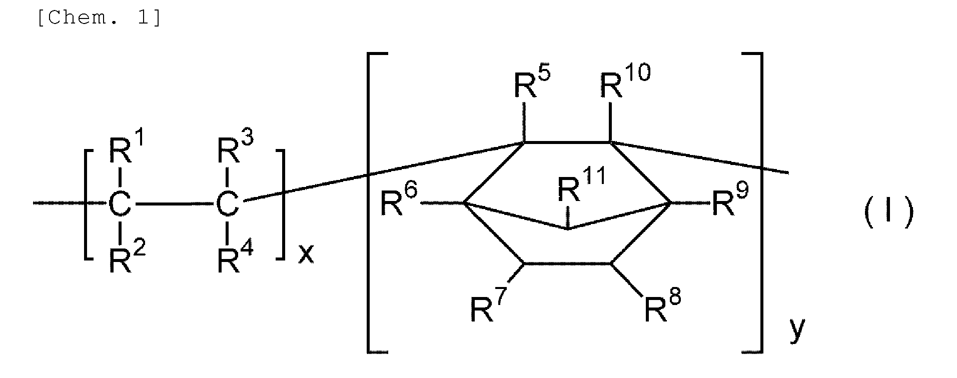

In the above-mentioned formula (I), R¹-R⁴ may be respectively the same or different and represent a hydrogen atom, a C₁-C₃ alkyl group, a hydroxy group, a methoxy group, an ethoxy group, a fluorine atom, a chlorine atom, or a bromine atom; while preferably R¹-R⁴ may be respectively the same or different and represent a hydrogen atom, a methyl group, or a fluorine atom; and more preferably all of R¹-R⁴ are hydrogen atoms. Also, R⁵-R¹¹ may be respectively the same or different and represent a hydrogen atom, a C₁-C₃ alkyl group (a methyl group, an ethyl group, or a propyl group), a hydroxy group, a methoxy group, an ethoxy group, a fluorine atom, a chlorine atom, or a bromine atom, or alternatively R⁷ and R⁸ represent together a C₄-C₆ bicyclo alkyl group which may be substituted with a methyl group, an ethyl group, a methoxy group, an ethoxy group, or a hydroxy group; while preferably R⁵-R¹¹ may be respectively the same or different and represent a hydrogen atom, a methyl group, a hydroxy group, a methoxy group, an ethoxy group, a fluorine atom, or a chlorine atom; and more preferably all of R⁵-R¹¹ are hydrogen atoms. "x" and "y" represent ratio of partial structure composing the ethylene system copolymer where x:y is 1:5 - 5:1, while 1:4 - 4:1 is preferable, 1:3 - 3:1 is more preferable, and 2:3-3:2 is further preferable. Another preferable embodiment of a hermetical container of the present invention is any one of the above-mentioned hermetical container, wherein the glass transition temperature of the resin composition is 140 °C - 180 °C.

The ampoule (20) was actually produced using various materials and experiments for breaking the joint were performed. However, when a material with excessively high crystallinity is used, the joint broke accompanied by generation of broken pieces. On the other hand, when a material with rich ductility or tractility is used, the breaking portion does not break successfully, and a so-called burr was generated. In the present invention, the hermetical container end is preferably composed only of the above-mentioned resin composition or includes such a resin composition as a main constituent, so that an appropriate hardness is realized and the joint can break smoothly while it is made possible to effectively prevent a situation where the broken pieces are generated. Also, by adopting such a resin composition, sterilization can be facilitated by an immersion in alcohol or an ultraviolet radiation, so that it becomes easy to handle. It is to be noted that for the above-mentioned resin composition the constituent monomers themselves are publicly known, so that by obtaining monomers respectively having double bonds to be mixed according to predetermined proportions and by additive polymerization thereof to be bonded the composition can be easily obtained. Also, the resin composition itself can be purchased from the market.

An example of the hermetical container end of the present invention applied to an ampoule for needleless syringe will now be described. As shown in Fig. 1, an ampoule usable as a needleless syringe is an ampoule provided with an ampoule main body (2) and a hermetical container end (10), wherein the ampoule main body (2) is preferably provided internally with a movable stopper whose diameter is almost the same as the internal surface of the ampoule main body (2). Also, a drug solution is filled preferably in a hermetically-closed state between the movable stopper and a sealed portion (sealed end of the front edge portion).

It is preferable that the ampoule main body (2) has a smooth cylindrical internal surface which enables a gasket (movable stopper) (40) to slide. As an amount of the drug solution to be contained in the ampoule, 1×10⁻¹ ml - 1x10 ml can be mentioned, while 1×10⁻¹ ml - 1 ml is acceptable. It is preferable that the ampoule main body (2) is provided with a shock-absorbing portion for absorbing an impact or an impact sound at a portion adjacent to the hermetical container end. It is to be noted that in order to move the movable stopper, the movable stopper is preferably coupled to a plunger or the like. The movable stopper may be composed of 1 or 2 flexible spheres, for example, for which the inner diameter of the ampoule matches the diameter of the sphere or the diameter of the sphere is 1.01 times - 1.1 times the inner diameter of the ampoule. It is to be noted that the movable stopper is preferably streamlined and the tip becomes narrower towards the ampoule end.

The ampoule of the present invention is used for a drug solution administering unit, for example. The drug solution administering unit is provided with an ampoule, an ampoule guide, and a release mechanism for releasing a drug solution within the ampoule.

### [Production method]

The hermetical container end, the ampoule and the ampoule guide of the present invention may be produced by a publicly known method for producing an ampoule or the like. Hereinafter, the ampoule of the present invention may be produced as an ampoule or the like provided with a hermetical container end. It is to be noted that molding methods include an injection molding (including a powder injection molding), an extrusion molding, and the like, while an injection molding using a predetermined metal mold can be used preferably. In this case, the above mentioned raw materials may be used as appropriate.

### [Usage]

Usage of the hermetical container end of the present invention will now be described. Basically, by applying a force to the front edge portion (1) from a lateral direction with respect to the central axis (5), the joint (4) breaks, and a head portion of the connection (3) which is circular truncated cone shaped is exposed and the space portion (13) of the connection (3) becomes an open end. This effect can be utilized so that the hermetical container end of the present invention can be applied, for example, to an ampoule for a needleless syringe or the like. Hereinafter, usage in such an embodiment of utilization will be described.

The ampoule (20) to be used as a needleless syringe is attached to the ampoule guide (holder: 30). In the state where the ampoule is attached to the ampoule guide, a force is applied to the front edge portion from a lateral direction, or the front edge portion is rotated, so that the joint breaks. Then, the ampoule attached to the ampoule guide is mounted on the needleless syringe main body. Thereafter, the open end of the needleless syringe is applied in close contact with the skin where the drug solution is to be administered.

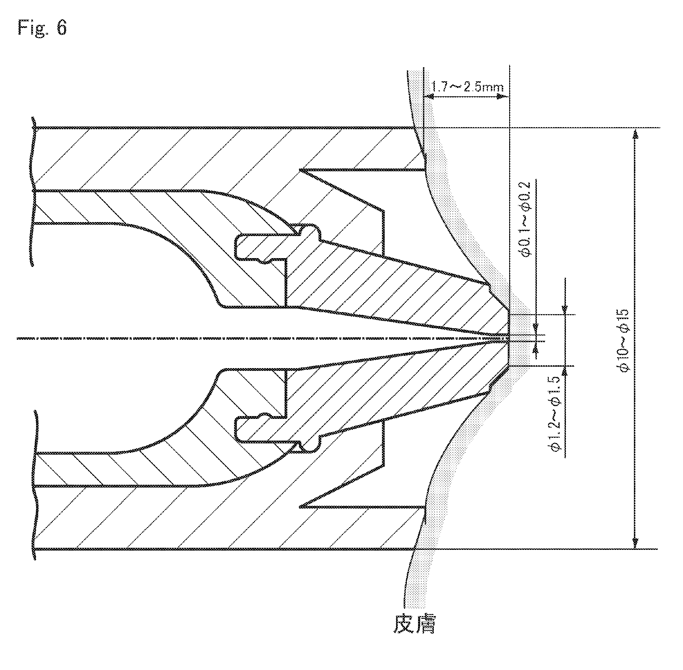

Fig. 6 is a schematic diagram showing a drug solution administering unit of the present invention as pressed against a skin. It is to be noted that numerical values within Fig. 6 are the values obtained upon actual designing.

When the button (51) is subsequently pressed, the gasket (40) included in the ampoule is pushed by the plunger and the like. The drug solution pushed by the movable stopper is emitted through the open end to be administered within the skin. As for the used needleless syringe unit, the ampoule (20) is removed from the ampoule guide (30) and the ampoule is disposed of.

As a needleless syringe, a publicly known needleless syringe can be used as appropriate. Specific needleless syringes include a needleless syringe described in Japanese Patent Application Laid-Open Publication No. 2003-093508, "a needleless syringe for injecting active principle comprising, from an upstream towards a downstream: a propelling system consisting of an impact wave generating device; a barrier having an upstream surface and a downstream surface, the downstream surface having at least one blind cavity where the active principle can be entered; and an application guide for applying the syringe to a skin of a patient to be treated, **characterized in that** on one hand the barrier is fixed to endure the impact wave while on the other hand the barrier guarantees to favorably propagate the impact wave" as described in Japanese Patent No. 3574433, and "a needleless syringe which injects a drug solution by plunging a piston having readily energized with ahead power in a latched state into the drug solution suctioned from an injection opening of a nozzle comprising: a front cylindrical portion having incorporated therein the nozzle having the injection opening; a rear cylindrical portion which is screwed into the front cylindrical portion and which has a recessed part provided in a longitudinal direction and a recessed part connected to the recessed part in the longitudinal direction on an opposite end to the injection opening to be provided in a circumferential direction of a rotational direction for suctioning of the drug solution; a ball holder integrally provided at the rear cylindrical portion; a plurality of balls in the ball holder; a piston driving rod provided with latching grooves for a part of each of the balls to move in and out and held by the rear cylindrical portion via the ball holder which has the balls included therein; a release switch having a protruding part which is inserted into the recessed part in the longitudinal direction or the recessed part in the circumferential direction of the rear cylindrical portion, and an abutting surface provided so as to abut on the balls in a state where the balls are in the latch grooves, wherein the protruding part can move along the recessed part in the longitudinal direction or the recessed part in the circumferential direction with respect to the rear cylindrical portion; anda compressed spring provided between the rear cylindrical portion and the release switch; **characterized in that** in a state where the balls are not included in the latch grooves, the balls abut on a near side of the abutting surface, so that the release switch is held resisting the compressed spring, and in a state where the balls are included in the latch grooves, the balls surpass the near side of the abutting surface to abut to the abutting surface and hold the piston driving rod, so that as for the release switch, the protruding part of the release switch is guided by the recessed part in the longitudinal direction to move to an end of the recessed part, while the ball holder and the ball rotate, so that the protruding part of the release switch enters the recessed part in the circumferential direction via the abutting surface abutting the balls, whereby a pushing operation of the release switch can be avoided" described in Japanese Patent No. 3560889 can be mentioned.

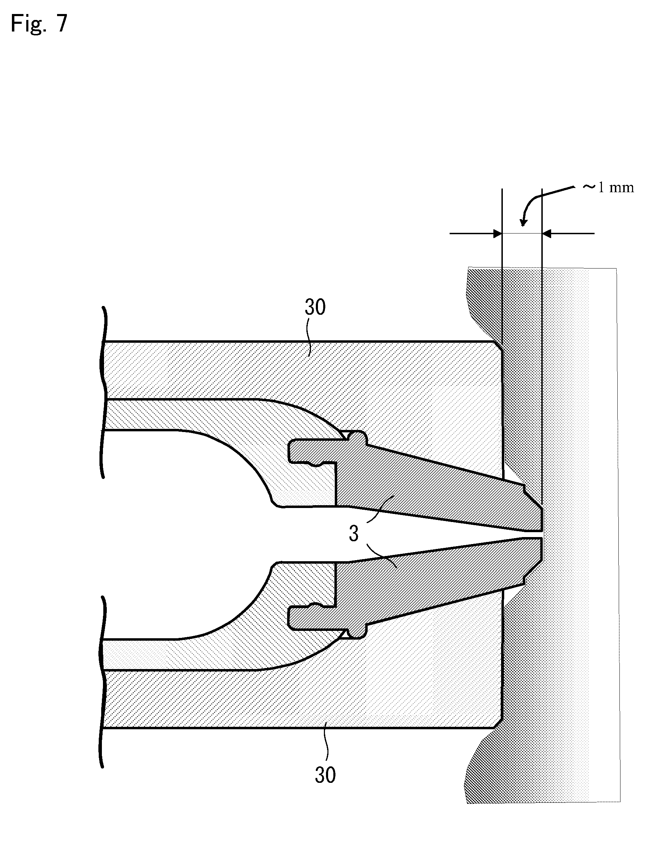

Fig.7 is a schematic diagram showing a drug solution administering unit of the present invention as pressed against a skin in a horizontal direction. The drug solution administering unit of the present invention can apply to all kinds of plane such as shown in figure 7 as well as the skin as shown in figure 6. In this case, the surface of the ampoule guide (30) which attaches to the plane may be plan as shown in the figure 7 and the position of the connection (3) may be higher than the position of the surface of the ampoule guide (30) by less than 1 mm.

### [Example 1]

Hereinafter, an example of an actual production of a drug solution administering unit will be described. As a resin for producing a hermetical container end, a resin represented by the formula (I) was synthesized, varying the composition ratio of "x" and "y" as appropriate, whereby the glass transition temperature was controlled. The resin actually used was one with all of R¹-R¹¹ being hydrogen atoms. Ethylene and bicyclo[2.2.1]hept-2-en were used as synthetic raw materials. Resins of various composition ratios were used to produce ampoules. Consequently, when the resins with the glass transition temperature of 140 °C - 180 °C (145 °C - 170 °C, 150 °C - 170, or °C150 °C - 160 °C is preferable) are used, ampoules with transparency and favorable cleavage were produced.

A molding die includes a pair of main-molding dies and a sub-molding die. The main-molding dies correspond to a contour of an ampoule for a needleless syringe, while the sub-molding die is used to mold a through-hole such as a space portion.

Firstly, the pair of the main-molding dies is set in a state separated from left to right facing each other in a molding position, and then moved to the lower side of a die for blow molding. A die having a function of pushing out a parison heated to a molding temperature from a push-out opening, and a function for infusing a pressurized gas such as a compressed air from an intake hole may be used.

Then, from the push-out opening of the die into the space between the main-molding dies, the parison composed of a hollow thermoplastic material semi-fused is pushed out. When the parison is pushed out for an appropriate length, the main-molding dies are united so that the parison is cut at the bottom portion of the main-molding dies, and then a compressed air is blown from the intake hole of the die into the sack-like parison to be inflated to fit the surface of the main-molding dies, so as to form the ampoule body. Then the die is taken out, and before the parison cools down, the sub-molding die is inserted to the center of the liquid infusing portion and the front edge portion in order to form the through-hole including the space portion. Lastly, by pulling of the main-molding dies and the sub-molding die, the ampoule can be produced.

Since only the hermetical container end was molded, a hermetical container end having a precise form was easily obtained.

Thereafter, three types of main body (2) were molded by using PET, methacrylic resin, and ABS resin, respectively. Also, ampoule guides were produced in the same way as the ampoule. **[Industrial Applicability]**

As described above, the ampoule of the present invention can be used as a hermetical container end or the like applied to an ampoule for a needleless syringe, so that the ampoule of the present invention can be preferably used in the field of medical equipment. It is obvious that the ampoule of the present invention can be used for other purposes than the needleless syringe in the field of medical equipment such as for a hermetical container end of a container for a single preparation unit in order to prepare an adequate amount of drug, or preferably used in the fields other than the medical equipment.

## Claims

1. An ampoule guide (30) comprising:
a hollow cylindrical guard (31) which is provided at a tipping portion,
the ampoule guide being capable of accommodating therein an ampoule (20).

2. The ampoule guide as claimed in claim 1, wherein when the ampoule (20) is inserted into the ampoule guide (30), a tip of the hollow cylindrical guard (31) is located on an inner side with respect to an open end of the ampoule (20).

3. A drug solution administering unit comprising:
an ampoule (20);
an ampoule guide (30) which is provided with a hollow cylindrical guard (31) at a tipping portion and which accommodates therein the ampoule (20); and
a gasket (40) which is set within the ampoule (20) for accommodating and pushing out a drug solution.

4. The drug solution administering unit as claimed in claim 3, wherein
the ampoule (20) comprises:
a front edge portion (1) which is removed when the ampoule (20) is used;
a main body (2) which can accommodate a drug solution;
a connection (3) which connects the front edge portion (1) and the main body (2); and
a joint (4) which joins the front edge portion (1) and the connection (3), and which is narrower than the front edge portion (1) and the connection (3);
the front edge portion(1), the joint (4), the connection (3) and the main body (2) being coaxially aligned to have a central axis (5), having sequential space portions (11, 14, 13, 12) extending from the main body (2) to a midway of the front edge portion (1), and the joint (4) breaking when a force is applied from a lateral direction with respect to the central axis (5), so that a head portion of the circular truncated cone shaped connection (3) is exposed and the space portion (13) of the connection (3) becomes an open end.

5. The drug solution administering unit as claimed in claim 4, wherein when the ampoule (20) is inserted into the ampoule guide (30), a tip of the hollow cylindrical guard (31) is located on an inner side with respect to an open end of the ampoule (20).

6. The drug solution administering unit as claimed in claim 4, wherein the ampoule guide (30) is provided with one or more notched parts (32) in a direction parallel to the central axis (5).

7. The drug solution administering unit as claimed in claim 4, wherein a plurality of projections (33) is provided in a direction parallel to the central axis (5), with projections that are taller than their surrounding projections among the plurality of projections being provided at angular intervals of any one of 60 degrees, 90 degrees, 120 degrees and 180 degrees.
